# EUROPÄISCHE PATENTSCHRIFT

(11) **EP 1 325 734 B1**
(45) Veröffentlichungstag und Bekanntmachung des Hinweises auf die Patenterteilung: **29.12.2004**
(21) Anmeldenummer: 02023866.3
(22) Anmeldetag: 24.10.2002
(51) Int. Cl.: A61K 7/40, A61K 31/19, C08L 23/06

(54) **Verfahren zur Herstellung von Ozoniden und Peroxiden von Fettsäuren oder deren Ester in Form einer streichfähigen Salbe**
Process for the preparation of ozone and peroxide derivatives of fatty acids or their esters in the form of a spreadable ointment
Procédé de préparation de dérivés ozonisés or peroxydés d'acides gras ou de leurs esters sous forme de pommade prête à l'emploi

(30) Priorität: 22.11.2001 DE 10157260
(43) Veröffentlichungstag der Anmeldung: 09.07.2003
(73) Patentinhaber: Pharmoxid Arznei GmbH & Co. KG, 76473 Iffezheim (DE)
(72) Erfinder: Bauer, Kurt, Dr. Prof., 79112 Freiburg (DE); Regenold, Jürgen, Dr., 79410 Badenweiler (DE); Viebahn-Hänsler, Renate, Dr., 76473 Iffezheim (DE)
(74) Vertreter: Flosdorff, Jürgen, Dr.

(56) Entgegenhaltungen:
- EP-A- 0 117 962
- EP-A- 0 291 838
- WO-A-01/37829
- FR-A- 2 730 636
- US-A- 4 545 990
- US-A- 4 906 617
- US-A- 5 110 583
- US-A- 5 204 093

## Beschreibung

Die Erfindung betrifft ein Verfahren zur Herstellung von Ozoniden und Peroxiden in Form von Fettsäuren oder deren Ester in Form einer streichfähigen Salbe sowie eine streichfähige, ohne Komplikationen topisch zu applizierende, stabile Salbe mit diesem Wirkstoff.

Die Herstellung von stabilen ozonisierten Ölen aus ungesättigten Pflanzenölen ist in der EP 0 235 528 B1 beschrieben, die hiermit in den Offenbarungsgehalt der vorliegenden Patentanmeldung einbezogen wird.

Pharmazeutische Zubereitungen, die Ozoniden oder Peroxiden von Fettsäuren enthalten, sind auch von EP 0 117 962, FR 2 730 636, und WO 0 137 829 her bekannt. Eine streichfähige Salbe, die andere Wirkstoffe enthält, ist aus EP 0 291 838 bekannt.

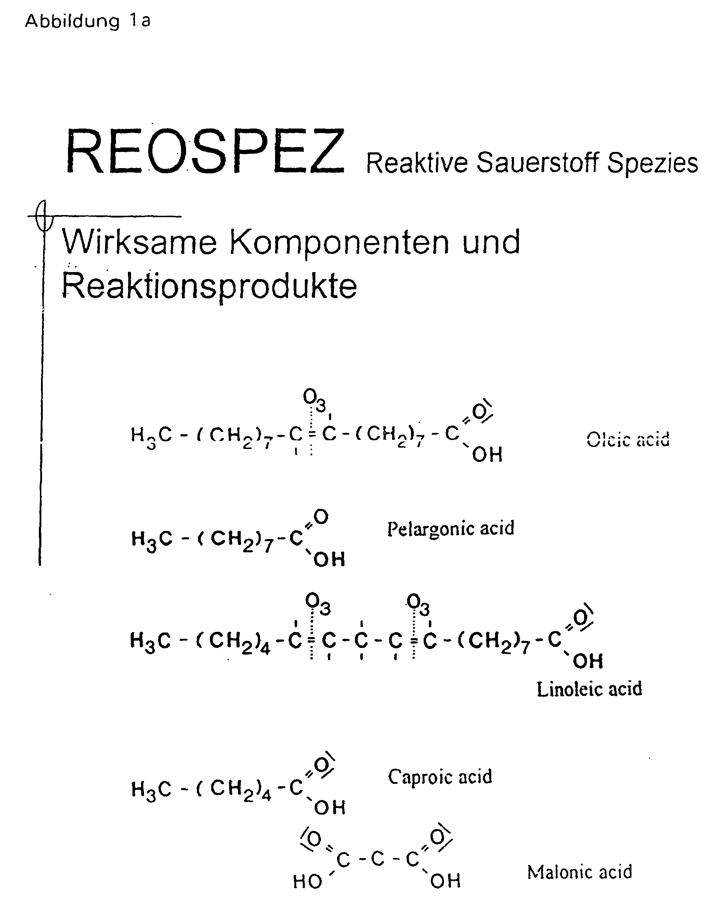

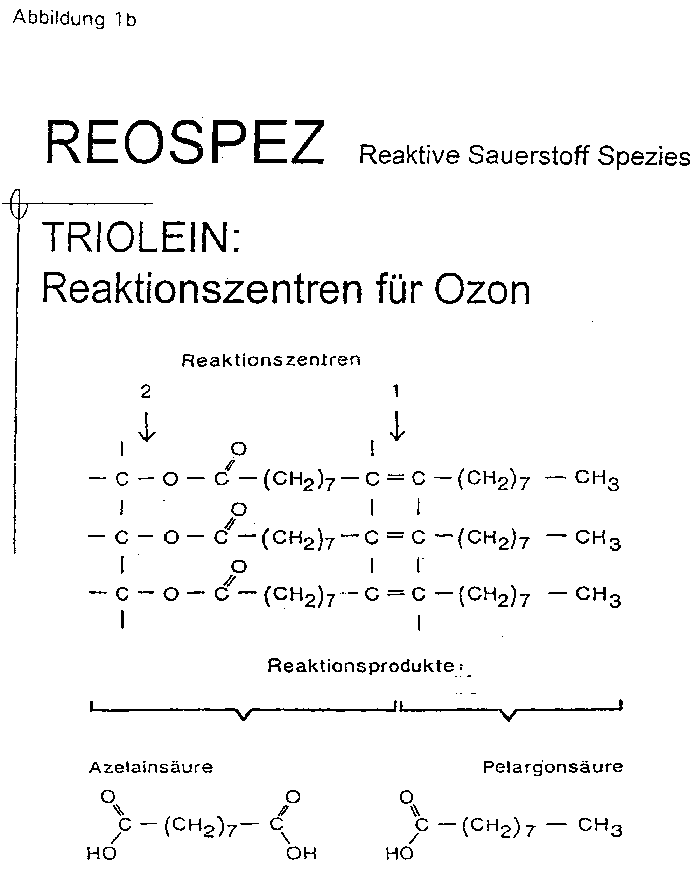

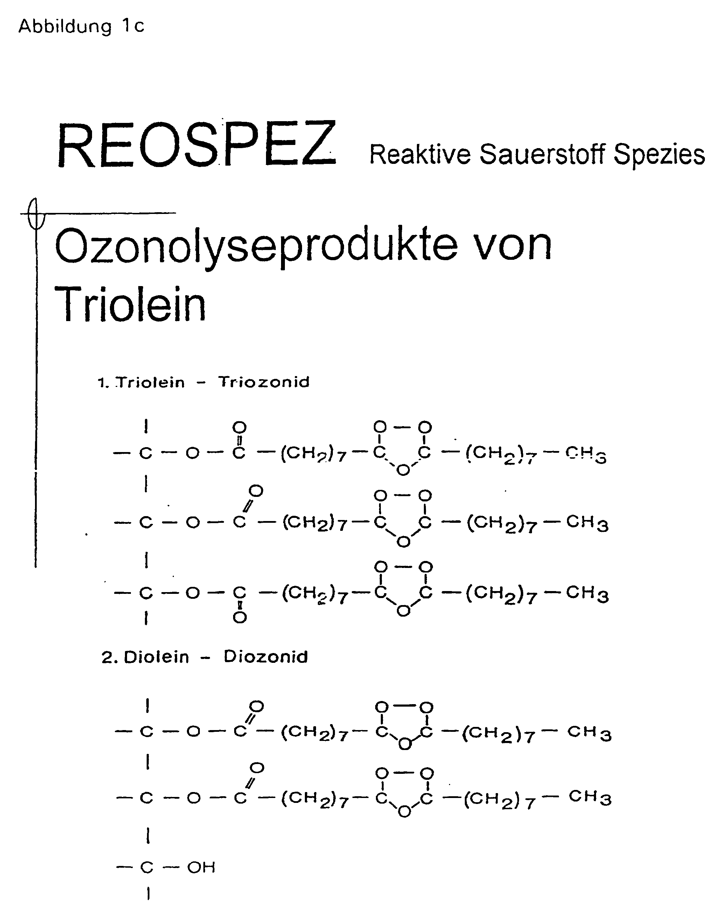

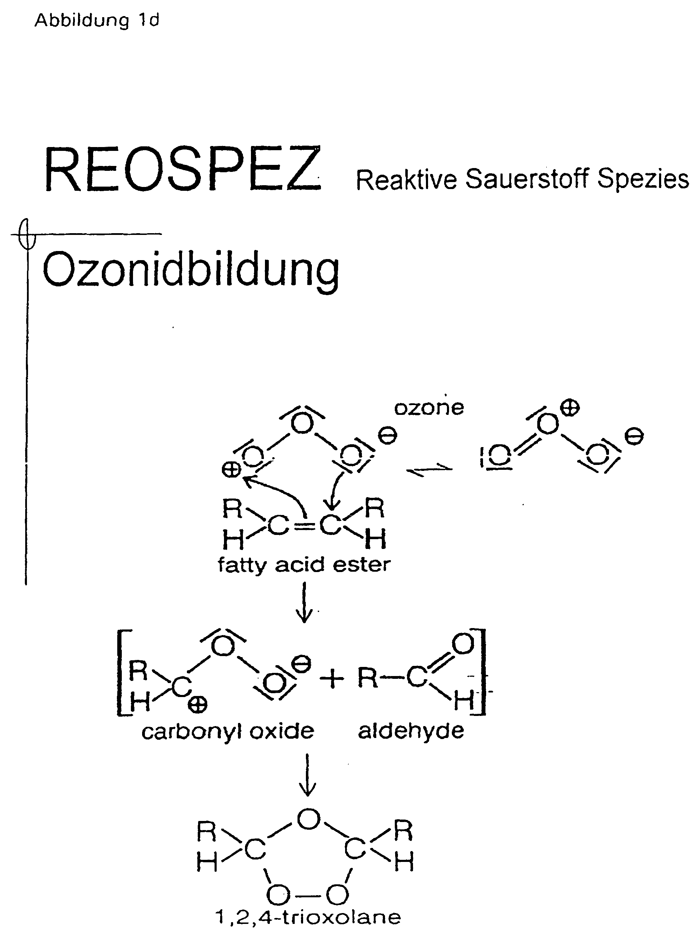

Bei der Umsetzung von Ozon mit ungesättigten Fettsäuren entstehen als therapeutisch wertvolle Wirkstoffe peroxidische Produkte, die eine fungizide und bakterizide Wirkung haben und insbesondere in der Dermatologie eingesetzt werden. Hierbei kommen in erster Linie die Ozonide von Olivenöl, Distelöl, Weizenkeimöl, Leinöl, Mandelöl, Sonnenblumenöl, Mohnöl, Sesamöl, Rizinusöl, Kroton- und Palmöl in Betracht. Am Beispiel von Triolein, Öl- und Linolsäure sind typische Ozonide in den Abbildungen 1a bis 1d dargestellt.

Die Ozonide und Peroxide von Fettsäuren oder deren Ester zerfallen in Abhängigkeit von ihrem Wassergehalt, der UV-Strahlung und der Temperatur allmählich unter Sauerstoffabgabe und verlieren ihre Wirksamkeit. Daher ist ihre Lagerfähigkeit insbesondere bei Zimmertemperatur begrenzt. Als Maß für die Konzentration und damit die Wirksamkeit der Wirksubstanz dient die Peroxidzahl (POZ), die nach einem validierten Verfahren gemessen und berechnet wird, wie dies in näheren Einzelheiten in der EP 0 235 528 B1 beschrieben ist.

Die Wirksubstanz wird zweckmäßigerweise in Form einer streichfähigen Salbe eingesetzt. Eine streichfähige Zubereitung läßt sich in Form einer Mischung mit aliphatischen Kohlenwasserstoffen herstellen, die gegenüber diesen Ozoniden und Peroxiden inert ist. Dabei erweist sich jedoch die physikalische Stabilität bereits bei Raumtemperaturen von 25 bis 30°C als unbefriedigend, da trotz der Inertness Phasentrennung und Farbveränderungen auftreten. Die chemische Stabilität bleibt insofern erhalten, als eine neuerliche Mischung der beiden Phasen bezüglich der Peroxidzahl der Spezifikation der Wirksubstanz entspricht. Auch die mikrobiologische Aktivität bleibt unverändert.

Dies ist in Tabelle 1 anhand einer Stabilitätsuntersuchung bei 30°C dargestellt: Bereits nach drei Monaten macht sich eine physikalische Instabilität durch Phasentrennung bemerkbar, wobei nach sechs Monaten zudem eine Gelbfärbung erfolgt. Das Mischungsverhältnis zwischen Wirkstoff und aliphatischen Kohlenwasserstoffer betrug bei diesem Versuch 1:1.

**Tabelle 1:**

| Wirksubstanz in einer 1:1- Mischung mit aliphatischen Kohlenwasserstoffen. Ergebnisse der Stabilität. | | | | |
|---|---|---|---|---|
| **Lagerungszeit bei 30 °C** | | **Beginn** | **3 Monate** | **6 Monate** |
| Parameter | Spezifikation | | | |
| **Beschreibung** | weiche, farblose bis schwach gelbliche Paste | Weiche, farblose bis schwach gelbliche Paste | Phasentrennung | Phasentrennung, gelblich |
| **Geruch** | typisch | typisch | typisch | typisch |
| **Stabilität** (Peroxidzahl) | > 100 | 199 | 149 | 121 |
| **Reinheit** | keine zusätzlichen Verbindungen | entspricht | entspricht | entspricht |

Der vorliegenden Erfindung liegt die Aufgabe zugrunde, eine streichfähige Salbe mit Ozoniden und/oder Peroxiden von Fettsäuren oder deren Ester als Wirksubstanz anzugeben, die eine ausreichende und zweckentsprechende physikalische Stabilität hat und über einen längeren Zeitraum ohne Phasentrennung und ohne Farbveränderung lagerfähig ist.

Außerdem soll ein Verfahren zur Herstellung einer derartigen Salbe angegeben werden.

Diese Aufgabe wird erfindungsgemäß durch die Merkmale der Patentansprüche 1 und 4 gelöst.

Vorteilhafte Weiterbildungen der Erfindung sind in den abhängigen Ansprüchen gekennzeichnet.

Im Verlaufe von Untersuchungen, die zur Lösung der oben genannten Aufgabe angestellt wurden, wurden Ozonide mit Paraffinen in unterschiedlichen Mengenverhältnisses gemischt. Dies führte zu keiner Verbesserung der physikalischen Stabilität.

Überraschend führte jedoch die Verwendung einer Salbengrundlage aus mit Polyethylen verdickten Paraffinölen zu erheblich verbesserten Stabilitätsdaten. Tabelle 3 gibt die Meßergebnisse bei 30°C bei den entsprechenden Lagerzeiten in Form der physikalischen Beschreibung und den Peroxidzahlen als Maß für die chemische Stabilität an. Demnach wurde nach drei Monaten und sechs Monaten Lagerzeit jeweils eine vollständige physikalische Stabilität ohne Phasentrennung und ohne Farbveränderung festgestellt.

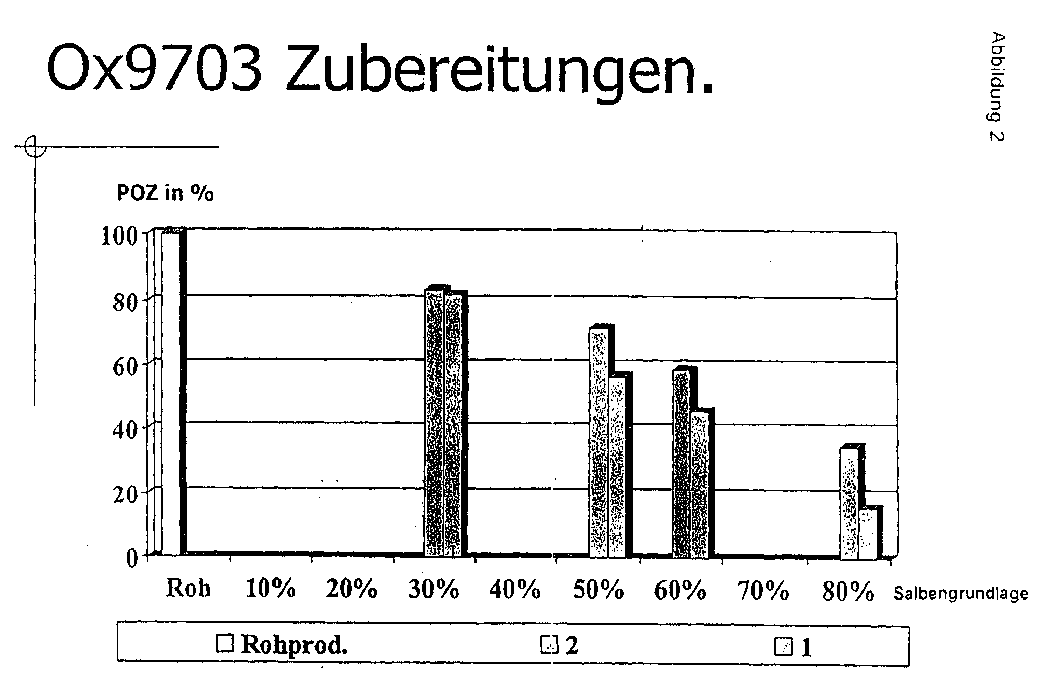

Überraschend wird durch eine Zubereitung mit mit Polyethylen verdickten Paraffinölen zudem der Gehalt an wirksamer Substanz erhöht, wie dies in Tabelle 2 und Abbildung 2 dargestellt ist. Wirkstoffmischungen mit 70, 50, 40 und 20% Wirkstoff wurden mit den beiden unterschiedlichen Salbengrundlagen zubereitet, nämlich mit unverdickten Paraffinen (1) und mit mit Polyethylen verdickten Paraffinen (2). Es wurde festgestellt, daß unabhängig von der Zusammensetzung der relative Anteil an Wirksubstanz in der Zubereitung (2) erhöht ist.

**Tabelle 2 :**

| Peroxidzahlen als Maß für den Gehalt der Wirksubstanz bei verschiedenen Mischungen der Wirksubstanz mit Paraffin (1) und polyethylenverdicktem Paraffin (2). | | |
|---|---|---|
| | Peroxidzahlen | |
| Mischungsverhältnis Wirksubstanz : Paraffin | 2 Polyethylenverdicktes Paraffin | 1 unverdicktes Paraffin |
| 70 : 30 | 83 | 82 |
| 50 : 50 | 71 | 56 |
| 60 : 40 | 59 | 45 |
| 20 : 80 | 35 | 16 |
| | | |

Tabelle 3 enthält ebenfalls Meßergebnisse der Peroxidzahlen als Maß für die chemische Stabilität der erfindungsgemäß hergestellten Salbe.

**Tabelle 3:**

| Wirksubstanz in einer 1:1- Mischung mit Polyethylen verdickten Paraffinen. Ergebnisse der Stabilität. | | | | |
|---|---|---|---|---|
| **Lagerungszeit bei 30** °C | | **Beginn** | **3 Monate** | **6 Monate** |
| Parameter | Spezifikation | | | |
| **Beschreibung** | weiche, farblose bis schwach gelbliche Paste | Weiche, farblose bis schwach gelbliche Paste | Weiche, farblose bis schwach gelbliche Paste | Weiche, farblose bis schwach gelbliche Paste |
| **Geruch** | typisch | typisch | typisch | typisch |
| **Stabilität** (Peroxidzahl) | > 100 | 187 | 141 | 143 |
| **Reinheit** | keine zusätzlichen Verbindungen | entspricht | entspricht | entspricht |

Die Salbenherstellung erfolgt in bekannter Weise durch Mischen in doppelwandigen und mit Kühlflüssigkeit kühlbaren Rührwerkskesseln, evtl. unter Vakuumbedingungen oder unter inerten Schutzgasen, bis das Gemisch ausreichend dispergiert und homogen ist. Dies ist der Fall, wenn die Ozonid- oder Peroxidzubereitungen sich in der Salbengrundlage mikroskopisch fein verteilt haben. Die Rührwerkskessel müssen außerdem in bekannter Weise hermetisch verschließbar sein, damit im Vakuum oder unter inerten Schutzgasen, z.B. Stickstoff, Argon, Kohlendioxid etc. gearbeitet werden kann, um eine jegliche unbeabsichtigte oder unerwünschte Einarbeitung von Luftsauerstoff oder Luftfeuchtigkeit in das Endprodukt auszuschließen.

Patentgemäße Produkte können auch, da sie aus Stabilitätsgründen außerordentlich extrem lipophil sind, im Bedarfsfall und nur für eine kurzzeitige Anwendung, beispielsweise von nicht länger als 3 Wochen, rezepturmäßig durch Zusatz von geeigneten Emulgatoren, z.B. Polyoxyethylensorbitanestern, hydrophilisiert werden oder es kann durch Zusatz von hydrophilen Gelbildnern die Haftfähigkeit verbessert werden.

Mit dem erfindungsgemäßen Verfahren werden gelartige, plastische, aber keinesfalls kristalline Gemische bzw. Produkte erzielt.

## Patentansprüche

1. Streichfähige Salbe mit Ozoniden und/oder Peroxiden von Fettsäuren oder deren Ester als Wirksubstanz, die mit einer Salbengrundlage gemischt ist,
**dadurch gekennzeichnet,**
**daß** diese Salbengrundlage aus mit Polyethylen verdickten Paraffinölen besteht, wobei das Polyethylen Molekulargewichte von 10.000 bis 30.000, vorzugsweise 21.000 hat.

2. Salbe nach Anspruch 1,
**dadurch gekennzeichnet,**
**daß** das Mischungsverhältnis der Wirksubstanz zur Salbengrundlage 70:30 bis 20 : 80 (Gew. %) beträgt.

3. Salbe nach Anspruch 1 oder 2,
**dadurch gekennzeichnet,**
**daß** die Ozonide von Olivenöl, Distelöl, Weizenkeimöl, Leinöl, Mandelöl, Sonnenblumenöl, Sesamöl, Rizinusöl, Kroton- oder Palmöl verwendet werden.

4. Verfahren zur Herstellung der Salbe nach einem der Ansprüche 1 bis 3,
**dadurch gekennzeichnet,**
**daß** das Polyethylen bei 130°C in der Salbengrundlage gelöst, anschließend langsam, innerhalb von 3 Stunden auf 100°C und schließlich durch Ausgießen in dünne Schichten auf Metallplatten sehr rasch auf 20°C abgekühlt wird.

## Claims

1. A spreadable ointment with ozone and/or peroxide derivatives of fatty acids or esters thereof as the active substance, which is mixed with an ointment base, **characterised in that** this ointment base consists of paraffin oils thickened with polyethylene, the polyethylene having molecular weights of 10,000 to 30,000, preferably 21,000.

2. An ointment as claimed in Claim 1, **characterised in that** the mixing ratio of the active substance to the ointment base is 70:30 to 20:80 (% by wt.).

3. An ointment as claimed in Claim 1 or 2, **characterised in that** the ozone derivatives of olive oil, thistle oil, wheat germ oil, linseed oil, almond oil, sunflower oil, sesame oil, castor oil, croton or palm oil are used.

4. A method of manufacturing the ointment as claimed in one of Claims 1 to 3, **characterised in that** the polyethylene is dissolved in the ointment base at 130°C, is then cooled slowly within 3 hours to 100°C and is finally cooled very rapidly to 20°C by pouring out in thin layers onto metal plates.

## Revendications

1. Pommade apte à être appliquée avec des dérivés ozonisés et/ou peroxydés d'acides gras ou de leurs esters comme substance active, qui est mélangée avec une base de pommade, **caractérisée en ce que** cette base de pommade est constituée d'huiles de paraffine épaissies avec du polyéthylène, où le polyéthylène présente des poids moléculaires de 10 000 à 30 000, de préférence de 21 000.

2. Pommade selon la revendication 1, **caractérisée en ce que** le rapport de mélange de la substance active à la base de la pommade est de 70:30 jusqu'à 20:80 (% en poids).

3. Pommade selon la revendication 1 ou 2, **caractérisée en ce que** les dérivés ozonisés d'huile d'olive, d'huile de chardon, d'huile de germes de blé, d'huile de lin, d'huile d'amandes, d'huile de tournesol, d'huile de sésame, d'huile de ricin, d'huile de croton ou de palme sont utilisés.

4. Procédé de fabrication de la pommade selon l'une des revendications 1 à 3, **caractérisé en ce que** le polyéthylène est dissous à 130°C dans la base de la pommade, est refroidi ensuite lentement, dans un délai de 3 heures à 100°C et enfin, par une coulée en couches minces sur des plaques métalliques, est refroidi très rapidement à 20°C.
